(19) **Europäisches Patentamt / European Patent Office / Office européen des brevets**

(11) **EP 1 917 977 B1**

(12) **EUROPEAN PATENT SPECIFICATION**

(45) Date of publication and mention
of the grant of the patent:
**28.12.2011 Bulletin 2011/52**

(21) Application number: **06796633.3**

(22) Date of filing: **22.08.2006**

(51) Int Cl.:
*A61K 39/395* (2006.01)  *A61P 9/00* (2006.01)
*A61P 9/04* (2006.01)  *A61P 31/12* (2006.01)
*A61P 31/14* (2006.01)  *A61P 31/16* (2006.01)
*A61P 31/18* (2006.01)  *A61P 31/20* (2006.01)
*A61P 31/22* (2006.01)  *A61P 35/02* (2006.01)

(86) International application number:
**PCT/JP2006/316419**

(87) International publication number:
**WO 2007/023815 (01.03.2007 Gazette 2007/09)**

(54) **THERAPEUTIC DRUG FOR HEART DISEASE AND VIRUS DISEASE**

THERAPEUTIKUM FÜR HERZKRANKHEIT UND VIRUSKRANKHEIT

MEDICAMENT THERAPEUTIQUE POUR MALADIE CARDIAQUE ET VIRUS

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK RS**

(30) Priority: **23.08.2005 JP 2005240803**

(43) Date of publication of application:
**07.05.2008 Bulletin 2008/19**

(73) Proprietor: **Matsumori, Akira
Minoh-shi
Osaka 562-0045 (JP)**

(72) Inventor: **Matsumori, Akira
Minoh-shi
Osaka 562-0045 (JP)**

(74) Representative: **Blodig, Wolfgang
Wächtershäuser & Hartz
Weinstrasse 8
80333 München (DE)**

(56) References cited:
WO-A-00/56770  WO-A-97/03696
WO-A-2004/058820  JP-A- 11 510 047
JP-A- 2002 542 768

- **REDEGELD F A ET AL: "Imunoglobulin-free light chains elicit immediate hypersensitivity-like responses" NATURE MEDICINE, NATURE PUBLISHING GROUP, NEW YORK, NY, US, vol. 8, no. 7, 1 July 2002 (2002-07-01), pages 694-701, XP002209461 ISSN: 1078-8956**
- **MEI S ET AL: "VASOACTIVE INTESTINAL PEPTIDE HYDROLYSIS BY ANTIBODY LIGHT CHAINS" JOURNAL OF BIOLOGICAL CHEMISTRY, AMERICAN SOCIETY OF BIOLOCHEMICAL BIOLOGISTS, BIRMINGHAM,; US, vol. 266, no. 24, 25 August 1991 (1991-08-25), pages 15571-15574, XP000872895 ISSN: 0021-9258**
- **UDA TAIZO ET AL: "Super catalytic antibody and antigenase." JOURNAL OF BIOSCIENCE AND BIOENGINEERING 2004, vol. 97, no. 3, 2004, pages 143-152, XP002503817 ISSN: 1389-1723**
- **KISHIMOTO C ET AL: "Immunoglobulin treatment prevents congestive heart failure in murine encephalomyocarditis viral myocarditis associated with reduction of inflammatory cytokines.", THE JOURNAL OF PHARMACOLOGY AND EXPERIMENTAL THERAPEUTICS NOV 2001 LNKD- PUBMED: 11602677, vol. 299, no. 2, November 2001 (2001-11), pages 645-651, ISSN: 0022-3565**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical field

The present invention relates to a therapeutic drug for heart failure and myocarditis.

Background Art

Heart failure is a pathological condition in which the heart fails to feed a sufficient amount of blood for maintaining the metabolism of tissues because of a disorder in its pumping action, and is caused by an ischemic heart disease such as myocardial infarction or angina pectoris, hypertension, cardiomyopathy, myocarditis, or a similar disease. Cardiomyopathy is a disease involving a disorder in cardiac muscle which provides the heart with a pumping action, and is classified into hypertrophic cardiomyopathy (thickening of cardiac muscle), dilated cardiomyopathy (thinning of cardiac muscle), restrictive cardiomyopathy (inhibition of dilatation), etc. Myocarditis is a disease involving inflammation of cardiac muscle, which is believed to be often caused by a virus.

At present, the means for treating heart failure, cardiomyopathy, myocarditis, and fulminant myocarditis is selected in accordance with the symptoms of these diseases. Examples of the therapeutic means include drug therapies employing a drug such as a heart stimulant, a diuretic agent, catecholamine, a vasodilator, an angiotensin-converting enzyme inhibitor, an angiotensin receptor antagonist, a β-blocker, an anti-arrhythmic agent, or an anti-inflammatory agent; and implantation of a cardiac pacemaker. In addition to these conventional therapeutic means, there is also demand for a therapeutic method based on a new strategy.

Meanwhile, a free immunoglobulin light chain (free light chain: FLC) which is dissociated from an immunoglobulin heavy chain is known to be excessively produced in pathologic states, particularly in myeloma. Such an FCL level increase is also reported in immunological diseases such as sarcoidosis (Non-Patent Document 1), Sjoegren's syndrome (Non-Patent Document 2), systemic lupus erythematosus (Non-Patent Document 3), multiple sclerosis (Non-Patent Documents 4 and 5), viral or bacterial meningitis (Non-Patent Document 4), AIDS (Non-Patent Documents 4 and 6), corneal grafting (Non-Patent Document 7), and rheumatoid arthritis (Non-Patent Documents 8 and 9).

Previous studies also revealed that the blood FLC level increases in such diseases as multiple sclerosis and rheumatoid arthritis, which are thought to be caused by mast cells (Non-Patent Documents 8 and 10). Another study revealed that the free kappa light chain level in blood is higher in atopic and non-atopic adult bronchitic asthma patients as compared with healthy subjects (Non-Patent Document 11). Peptide F991, which is formed of nine amino acids and serves as an FLC antagonist, is reported to be effectively suppressing respiratory obstruction and pneumonitis in non-atopic asthma model mice (Non-Patent Document 11). Also, FLC plays an important role in the onset of mast-cell-dependent contact allergy, and F991 (an FLC antagonist) can prevent the onset thereof (Non-Patent Document 12). However, there have never been elucidated effects of FLC on viral diseases, heart failure, cardiomyopathy, myocarditis, fulminant myocarditis, and other diseases.

Non-Patent Document 1: Romer F.K. et al., Eur. J. Respir. Dis. 1984; 65: 292-295
Non-Patent Document 2: Moutsopoulos H.M. et al., J. Immunol. 1983; 130: 2663-2665
Non-Patent Document 3: Hirohata et al., J. Rheumatol. 1986; 13: 715-721
Non-Patent Document 4: Fagnart O.C. et al., J. Neuroimmunol. 1988; 19: 119-132
Non-Patent Document 5: Lamers K.J. et al., J. Neuroimmunol. 1995; 19: 119-132
Non-Patent Document 6: Contini C. et al., J. Neuroimmunol. 2000; 108: 221-226
Non-Patent Document 7: Solling K. et al., Scan. J. Clin. Lab. Invest. 1978; 38: 369-373
Non-Patent Document 8: Solling K. et al., Acta Med. Scand. 1981; 209: 473-477
Non-Patent Document 9: Cooper A. et al., Ann. Rheum. Dis. 1968; 27: 537-543
Non-Patent Document 10: Lee D.M. et al., Science 2002; 297: 1689-1692
Non-Patent Document 11: Kraneveld A.D. et al., Proc. Natl. Acad. Sci. USA. 2005; 102: 1578-1583
Non-Patent Document 12: Redegeld F.A. et al., Nat. Med. 2002; 8: 694-701

Kishimoto et al., J. Pharmacol. Exp. Ther. (2001) 299, 645-651 relates to the treatment of congestive heart failure by an immunoglobulin.

Disclosure of the Invention

Problems to be Solved by the Invention

An object of the present invention is to provide a new therapeutic drug for heart failure, and myocarditis.

Means for Solving the Problems

In the aforementioned bronchitic asthma, the free kappa light chain level increases, but the free lambda light chain does not increase. Since an FLC antagonist is effective for bronchitic asthma, FLC is conceived to aggravate the disease. Meanwhile, the present inventor previously found that the free kappa light chain level and free lambda light chain level in blood increase in patients of a viral disease, heart failure, cardiomyopathy, or myocarditis, and that the lambda chain level increases more as compared with the kappa chain level and the kappa/lambda ratio decreases in these patients (Japanese Patent Application No. 2005-114350). The further study of the inventor has revealed that the cardiac muscle lesion can be significantly mitigated and the survival rate can be significantly improve by administration of a free immunoglobulin light chain in encephalomyocarditis viral myocarditis (heart failure) model mice. Thereby the inventor has found that the free immunoglobulin light chain is useful as a therapeutic drug for heart diseases. The inventor has also revealed that the HCV antigen level of HCV-infected cells can be significantly reduced through administration of a free immunoglobulin light chain to the cells, and thereby found that the free immunoglobulin light chain is useful as a therapeutic drug for viral diseases.

Accordingly, the present invention provides a use of a free immunoglobulin light chain as defined in claim 1 for producing a therapeutic drug for heart failure or myocarditis. Also provided is the free immunoglobulin light chain for the use in the therapy of heart disease as defined in claim 5.

Effects of the Invention

According to the present invention, lesions and survival rate of patients of heart failure, and myocarditis, can be improved. Thus, the invention can provide a new therapeutic means for the treatment of these diseases. It is also described herein that the virus antigen level of patients of various viral diseases such as viral hepatitis can be reduced, to thereby treat the viral diseases. The treatment of pericarditis, and other viral diseases, is thus also described.

Brief Description of the Drawings

[Fig. 1] A graph showing changes in survival rate of encephalomyocarditis viral myocarditis (heart failure) models through administration of a free kappa light chain.
[Fig. 2] Photographs showing an antiviral effect of a free immunoglobulin kappa light chain on peripheral blood mononuclear leucocytes of patients infected with hepatitis C virus.
[Fig. 3] Photographs showing an antiviral effect of a free immunoglobulin kappa light chain on THP-1 cells infected with hepatitis C virus.

Best Modes for Carrying Out the Invention

The free immunoglobulin light chains (FLCs) employed in the therapeutic drug of the present invention are kappa chain. A mixture of a kappa chain and a lambda chain may also be employed. When such a mixture is employed, a mixture having a high kappa/lambda ratio of 2 or higher is used.
These FLCs may be obtained through, for example, separating them from urine or blood of human subjects, gene recombination, or collecting them from myeloma cells. FLCs may be separated from urine or blood samples by means of, for example, electrophoresis or gel filtration (Solomon A., Methods Enzymol. 1985; 116:101-121).
As shown in the Referential Example and the Examples hereinbelow, in heart failure, cardiomyopathy, myocarditis, fulminant myocarditis, and viral diseases, the free kappa light chain blood level and the free lambda light chain level in blood increase, and the kappa/lambda ratio decreases. When a free kappa light chain is administered to viral myocarditis (and heart failure) models, survival rate and the cardiac muscle lesion of the models can be significantly improved. Therefore, an FLC, particularly a kappa chain, is a useful therapeutic drug for heart diseases such as heart failure, cardiomyopathy, myocarditis, and fulminant myocarditis, and for viral diseases. When a free kappa light chain is administered to virus-infected cells, the virus antigen level of the cells is reduced. Therefore, an FLC, particularly a kappa chain, is a useful therapeutic drug for various viral diseases. Herein, these viral diseases include diseases caused by a pathogenic virus belonging to DNA viruses or RNA viruses. Examples of such pathogenic viruses include the following:

DNA viruses: Poxvirus, Herpesvirus, Adenovirus, and Parvovirus; and
RNA viruses: Reovirus, Togavirus, Coronavirus, Rhabdovirus, Paramyxovirus, Orthomyxovirus, Bunyavirus, Arenavirus, Retrovirus, Picornavirus, and Calicivirus.

In particular, the therapeutic drug of the present invention is preferably employed for the treatment of a patient suffering from viral myocarditis caused by an RNA virus or a hepatitis virus, or suffering from a viral disease induced by the viral myocarditis, and prevention of such diseases. Examples of particularly preferred target RNA viruses include Orthomyxovirus and Picornavirus.

Specific examples of viral diseases induced by viral myocarditis include viral hepatitis (type A, type B, type C, type E, type G, and type TTV), adenovirus infections, influenza, viral pneumonia, viral bronchitis, herpes infections (herpes simplex, EB virus (infectious mononucleosis), and herpes zoster), polio, AIDS (HIV infections), adult T cell leukemia (ATL), papilloma, measles, rubella, exanthema subitum, infectious erytheme, viral encephalitis, viral meningitis, cytomegalovirus infections, mumpus, chicken pox, lyssa, viral enteritis, viral pericarditis, Coxsackievirus infections, echovirus infections, hemorrhagic fever with renal syndrome, and Lassa fever. Among these viral diseases, the therapeutic drug of the present invention is preferably applied to viral hepatitis (type A, type B, type C, type E, type G, and type TTV), adenovirus infections, influenza, herpes infections, viral encephalitis, cytomegalovirus infections, viral enteritis, and viral pericarditis. The therapeutic drug described herein is also effective for the treatment of these heart diseases and viral diseases showing a reduced FLC kappa/lambda ratio.

The therapeutic drug of the present invention may be administered, for example, intravenously, subcutaneously, intramuscularly, or intraperitoneally. Examples of drug compositions suitable for these administration routes include injection solution, lyophilized drug products, and powders. For producing these compositions, an FLC or a constitutive polypeptide thereof may be mixed with a solvent, a solution adjuvant, a stabilizer, etc.

The dose of the therapeutic drug of the present invention, which varies depending on the type, symptoms, etc. of the disease, is preferably 0.1 mg to 10 g/day/adult, as reduced to free kappa light chain.

Examples

The present invention will next be described in more detail by way of Referential Example and Examples, which should not be construed as limiting the invention thereto.

Referential Example 1

(1) Serum specimens

Serum specimens tested in this experiment were collected from healthy volunteers, heart failure patients, cardiomyopathy patients, and myocarditis patients.

(2) FLC-specific monoclonal antibody

An FLC-specific monoclonal antibody was produced through immunizing BALB/c mice with FLC (product of Bethyl laboratories, Montgomery, TX) (J. Immunol. Methods, 275, 9-17. (2003)). An $F(ab')_2$ fragment of the produced antibody was labeled with horseradish peroxidase (HRP) (J. Histochem. Cytochem., 22, 1084-91. (1974)).

(3) FLC assay

FLC assay was performed according to literature procedures (J. Immunol. Methods, 2003, 275, 9-17). Specifically, a specimen or a standard concentration solution (each 100 $\mu$L) was added to a 96-well microplate (Nunc) coated with the FLC-specific monoclonal antibody, followed by reaction at room temperature for two hours. After washing, an HRP labeled anti-kappa light chain antibody and an HRP-labeled anti-lambda light chain antibody (each 100 $\mu$L) serving as secondary antibodies were added to the microplate, followed by reaction at 37°C for 30 minutes. After reaction, the microplate was washed with PBS containing 0.05% Tween 20, and the reactions were allowed to develop color by dispensing to each well a color developing solution (OPD, product of Sigma) (each 100 $\mu$L). The color development was terminated with 1N sulfuric acid, and absorbance at 492 nm was measured. In the assay, 50mM Tris-HCl (pH 7.5) containing 1% bovine serum albumin was employed as a buffer.

(4) Assay results

(4-1) Table 1 shows the results of FLC assay for heart failure patients (63 cases) (from Dec. 2002 to Nov. 2004).
[Table 1]

|  | n | kappa (mg/L) | lambda (mg/L) | kappa/lambda |
|---|---|---|---|---|
| Heart failure | 63 | 40.8±39.2* | 93.2±67.9* | 0.38±0.22* |
| Healthy subjects | 17 | 27.9±5.3 | 43.6±8.8 | 0.66±0.16 |
| Mean ± standard deviation, *p<0.0001 | | | | |

(4-2) Of all the patients, 17 patients who had undergo measurement of NT-proBNP level and SCF level were investigated in terms of correlation with FLC levels. The results are shown in Table 2.

[Table 2]

| X | NT-proBNP(y) | | | SCF(y) | | |
|---|---|---|---|---|---|---|
|  |  | $r^2$ | P |  | $r^2$ | p |
| Kappa | y=280x-4920 | 0.511 | 0.0013 | y=12.9x+381 | 0.3518 | 0.012 |
| Lambda | y=51.6x-455 | 0.806 | <0.0001 | y=1.72x+642 | 0.289 | 0.0259 |
| Kappa/lambda | y=-32600x+18900 | 0.564 | 0.0005 |  | 0.0641 | 0.327 |

Correlation of NT-proBNP with the FLC level in 63 patients was determined to be expressed by the following equation:

NT-proBNP

$$y=206x-12200, \quad r^2=0.481, \quad p<0.0001.$$

(4-3) Serum FLC levels determined in a multi-center clinical trial on myocarditis treatment are shown below.
(4-3-1) Table 3 shows the serum FLC levels as measured all the myocarditis patients.

[Table 3]

|  | n | kappa (mg/L) | lambda (mg/L) | kappa/lambda |
|---|---|---|---|---|
| Myocarditis | 1,318 | 37.6±19.2** | 72.4±124.2* | 0.64±0.32 |
| Healthy subjects | 17 | 27.9±5.3 | 43.6±8.8 | 0.66±0.16 |
| Mean ± standard deviation, *p<0.0005, **p<0.0001 | | | | |

(4-3-2) Table 4 shows the effect of infection with hepatitis C virus (HCV) on FLC levels.

[Table 4]

|  | n | kappa (mg/L) | lambda (mg/L) | kappa/lambda |
|---|---|---|---|---|
| HCV (+) | 42 | 30.6±14.8* | 173.1±37.1* | 0.27±0.22* |
| HCV (-) | 1,276 | 37.9±19.3* | 69.1±81.7* | 0.66±0.31 |
| Healthy subjects | 17 | 27.9±5.3 | 43.6±8.8 | 0.66±0.16 |
| Mean ± standard deviation, *p<0.0001 with respect to healthy subject, HCV (+,-), p<0.0001 | | | | |

(4-3-3) Table 5 shows the correlation with NT-proBNP.

[Table 5]

|  | NT-pro-BNP (y) | | |
|  | X | $r^2$ | p |
| --- | --- | --- | --- |
| Kappa | y=64.8x+311 | 0.0197 | <0.0001 |
| Lambda | y=6.58x+2270 | 0.008496 | 0.0008 |
| Kappa/lambda | y=-2255x+4202 | 0.0062 | 0.0044 |

(4-3-4) Table 6 shows the FLC levels of cardiomyopathy patients who are positive for hepatitis C virus antibody.

[Table 6]

|  | n | kappa | lambda | kappa/lambda |
| --- | --- | --- | --- | --- |
| Hepatitis C virus 5 antibody (+) |  | 42.5±27.2* | 79.9±74.0* | 0.558±0.338 |
| Healthy subjects | 17 | 27.9±5.3 | 43.6±8.8 | 0.661±0.162 |

*p<0.05

(4-3-5) Relationship between FLC levels and severity of heart failure

The tested subjects were classified into two categories in terms of severity (light (grades I + II) and grave (grades III + IV)). Each grade denotes a heart function classification on the basis of subjective symptoms graded by the New York Heart Association (NYHA). The results are shown in Table 7.

[Table 7]

|  | NYHA heart function classification | | |
|  | Light (grades I + II) (n=17) | Grave (grades III + IV) (n=63) | p |
| --- | --- | --- | --- |
| Kappa | 32.0±15.4 | 40.8±39.2 | NS |
| Lambda | 55.0±44.0 | 93.2±67.9 | 0.03 |
| Kappa/lambda | 0.67±0.31 | 0.46±0.14 | 0.0001 |

(5) Results

As is clear from Tables 1 to 7, in all the tested patients suffering from heart failure, cardiomyopathy, myocarditis, or a viral disease, the free kappa light chain level and the free lambda light chain level significantly increased, while the kappa/lambda ratio significantly decreased.

Example 1 (Effect of administration of an FLC on encephalomyocarditis viral myocarditis (heart failure) model)

(Procedure)

Encephalomyocarditis virus (1-plaque-forming unit) was injected to the abdominal cavity of each of 4-week-old male DBA/2 mice, and a human free kappa light chain (product of Bethyl laboratories) (10 μg/mouse/day) was subcutaneously injected to the mouse. Instead of the kappa light chain, physiological saline (400 μL) was subcutaneously injected to the mice of a control group. On day 5, the heart was removed from each mouse, and the left ventricle was cut in a horizontal direction, fixed with 10% formalin to prepare paraffin sections. The sections were subjected to hematoxylin and eosin staining and Masson's trichrome staining.

The area of a lesion in cardiac muscle of each mouse was analyzed by means of microanalyzer graphic analysis software (product of Finggal Link Co., Ltd.), whereby the percent lesion area of each cardiac muscle sample was calculated. The obtained data were statistically processed through analysis of variance, and multi-group comparison was performed through the Fischer LSD method.

Survival rate on day 14 was obtained through the Kaplan-Meier viability analysis, and significance was assessed through the Logrank test.

(Results)

(1) Area of cardiac muscle lesion:

[Table 8]

|  | Cardiac muscle lesion |
| --- | --- |
| Control group (n=8) | 39.0±3.8% (mean±standard error) |
| Human free kappa light chain-administered group (n=9) | 18.9+2.3% (p<0.01, vs. control group) |

As is clear from Table 8, cardiac muscle lesion was significantly mitigated in the human free kappa light chain administration groups.

(2) Survival rate

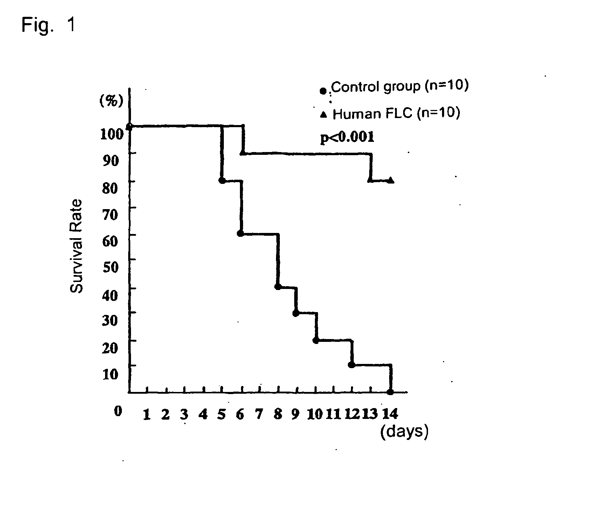

As shown in Fig. 1, all 10 of the tested mice in the control group died within 14 days after injection of encephalomyocarditis virus. In contrast, only two mice died in each human free kappa light chain administration group. Therefore, survival rate of the administration group was significantly improved.

Example 2

(Procedure)

The virus level of the heart on day 5 after injection of the virus was determined through the plaque quantitation method employing FL cells (Matsumori A, et al., Circulation. 1985: 71: 834-839).

(Results)

The virus level of the heart was found to be $(1.7\pm0.5)\times10^6$ PFU/g (n=8) in the control group. In contrast, in the human free kappa light chain administration groups, the virus level in the heart was significantly reduced to $(0.12\pm0.05)\times10^6$ PFU/g (n=8) (p<0.005).
Thus, a free kappa light chain was found to suppress proliferation of encephalomyocarditis virus in the heart.

Example 3

Studies on an antiviral effect of an FLC employing peripheral blood leukocytes infected with hepatitis C virus (HCV)

(Procedure)

Peripheral blood was collected from patients infected with HCV, from which, mononuclear leucocytes were selectively separated through density gradient centrifugation using Ficoll pack liquid (product of Amersham, U.S.A.). The mononuclear leucocytes were dispersed in an RPMI 1640 medium containing 10% fetal bovine serum to a concentration of $1 \times 10^6$ cells/mL. The thus-produced suspension was added to a 24-well plate at 1 mL/well.
An FLC (1 $\mu$g) was added to each well of the plate, and cultured at 37°C under 5% $CO_2$ conditions for five days. After completion of culturing, cultured mononuclear leucocytes were collected from each well and applied onto a slide, followed by drying and fixing with ethanol. To the fixed samples, a mouse monoclonal antibody to a core domain protein of HCV (Core A-2 antibody, product of Institute of Immunology Co., Ltd., Takahashi, K., et al. , Gen. Virol., 73: 667-672, 1992) (hereinafter referred to as "core antibody") was added as a primary antibody in an amount of 0.2 mL (20 $\mu$g/mL), followed by incubation overnight at 4°C. Subsequently, immunostaining was performed by use of a Bectastein ABC kit for mouse IgG (Burlingame, CA, U.S.A.) according to the instruction manual of the kit, wherein color was allowed to develop with DAB (diaminobenzidine).

(Results)

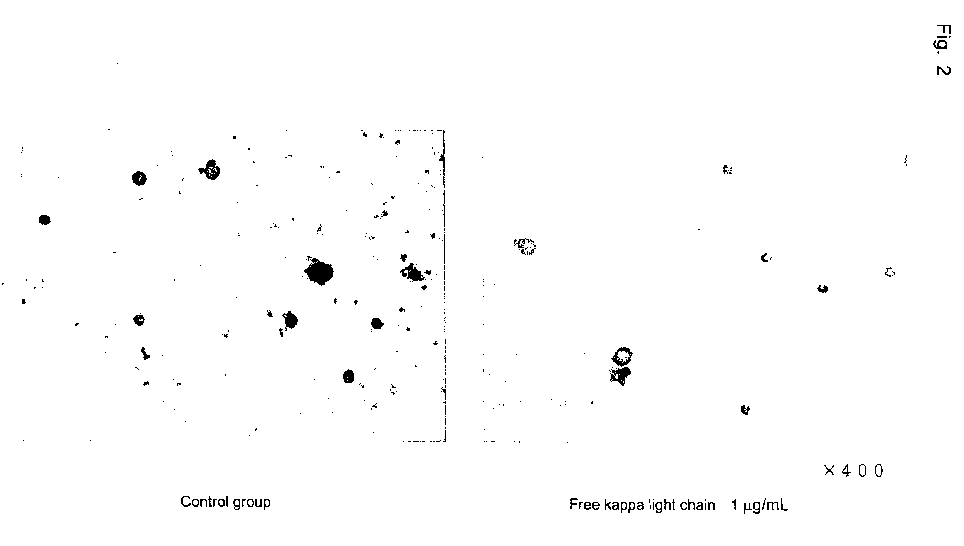

Fig. 2 shows the results. In Fig. 2, color density as obtained from immunostaining of samples of the free kappa light chain-added group (right) was considerably lowered, as compared with the control groups (left). Thus, the HCV antigen level of the mononuclear leucocytes was found to decrease by the action of a free kappa light chain.

Example 4

Studies on antiviral effect of FLC employing HCV-infected monocytes

(Procedure)

Monocytes strain THP-1 (American Type Culture Collection, Manassas, VA, VSA) were dispersed in an RPMI medium containing 10% fetal bovine serum to a concentration of $1 \times 10^6$ cells/mL and cultured in a 24-well plate. To the cultures, serum of a patient infected with HCV (HCV assay: 225 KIU/mL) and a free kappa light chain (1 $\mu$g) were added, followed by culturing at 37°C under 5% $CO_2$ conditions for five days. Subsequently, cultured THP-1 cells were collected from each well, and immunostaining was performed by use of a core antibody in a manner similar to that of Example 3.

(Results)

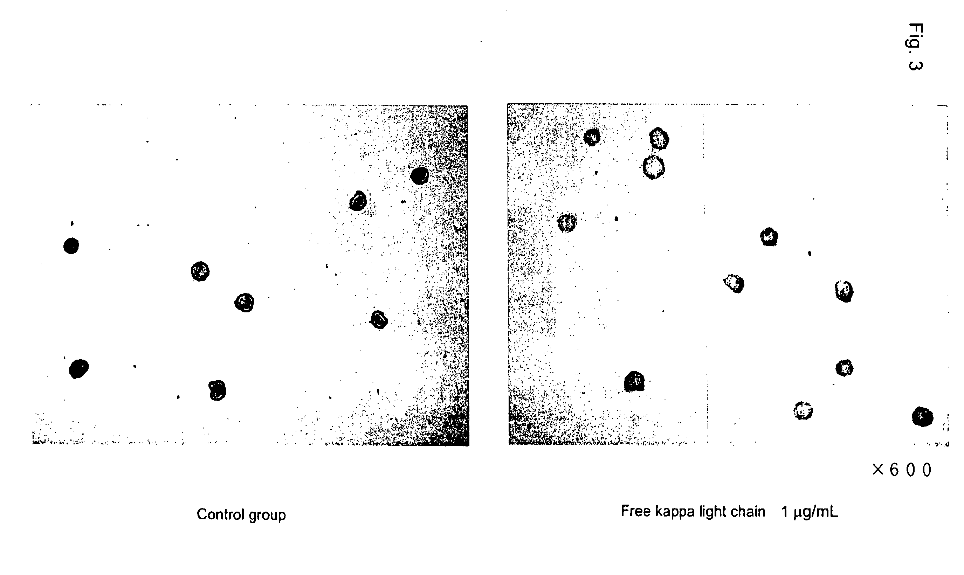

Fig. 3 shows the immunostaining results of THP-1 cells cultured with serum of HCV-infected patient and those of THP-1 cells cultured in a similar medium to which a free kappa light chain has been added. As is clear from Fig. 3, the immunostaining image (right) of THP-1 cells to which a free kappa light chain has been added is a less positive image, as compared with the immunostaining image (left) of THP-1 cells to which no free kappa light chain has been added. Thus, the HCV antigen level of the monocytes was found to decrease by the action of a free kappa light chain.

**Claims**

1. Use of a free immunoglobulin light chain for producing a therapeutic drug for heart disease,
   wherein the free immunoglobulin light chain is a human kappa chain or a mixture of a human kappa chain and a human lambda chain, wherein the mixture has a kappa/lambda ratio of 2 or higher, and
   wherein the heart disease is heart failure or myocarditis.

2. The use as described in claim 1, wherein the free immunoglobulin light chain is a kappa chain.

3. The use as described in claim 1 or 2, wherein the heart disease is myocarditis.

4. The use as described in claim 3, wherein the myocarditis is encephalomyocarditis viral myocarditis.

5. A free immunoglobulin light chain for use in the therapy of heart disease,
   wherein the free immunoglobulin light chain is a human kappa chain or a mixture of a human kappa chain and a human lambda chain, wherein the mixture has a kappa/lambda ratio of 2 or higher, and
   wherein the heart disease is heart failure or myocarditis.

6. The free immunoglobulin light chain for use according to claim 5, wherein the free immunoglobulin light chain is a kappa chain.

7. The free immunoglobulin light chain according to claim 5 or 6, wherein the heart disease is myocarditis.

8. The free immunoglobulin light chain according to claim 7, wherein the myocarditis is encephalomyocarditis viral myocarditis.

**Patentansprüche**

1. Verwendung einer freien leichten Kette eines Immunoglobulins zur Herstellung eines therapeutischen Arzneimittels für eine Herzerkrankung, worin die freie leichte Kette eines Immunoglobulins eine humane kappa-Kette oder eine Mischung aus einer humanen kappa-Kette und einer humanen lambda-Kette ist, wobei die Mischung ein kappallambda-Verhältnis von 2 oder größer aufweist und worin die Herzerkrankung Herzversagen (Herzinsuffizienz) oder Myocarditis ist.

**2.** Die Verwendung wie in Anspruch 1 beschrieben, worin die freie leichte Kette eines Immunoglobulins eine kappa-Kette ist.

**3.** Die Verwendung wie in Anspruch 1 oder 2 beschrieben, wobei die Herzerkrankung Myocarditis ist.

**4.** Die Verwendung wie in Anspruch 3 beschrieben, worin die Myocarditis Encephalomyocarditis virale Myocarditis ist.

**5.** Freie leichte Kette eines Immunoglobulins zur Verwendung in der Therapie einer Herzerkrankung, worin die freie leichte Kette eines Immunoglobulins eine humane kappa-Kette oder eine Mischung aus einer humanen kappa-Kette und einer humanen lambda-Kette ist, wobei die Mischung ein kappa/lambda-Verhältnis von 2 oder größer aufweist und worin die Herzerkrankung Herzversagen (Herzinsuffizienz) oder Myocarditis ist.

**6.** Die freie leichte Kette eines Immunoglobulins zur Verwendung gemäß Anspruch 5, worin die freie leichte Kette eines Immunoglobulins eine kappa-Kette ist.

**7.** Die freie leichte Kette eines Immunoglobulins gemäß Anspruch 5 oder 6, worin die Herzerkrankung Myocarditis ist.

**8.** Die freie leichte Kette eines Immunoglobulins gemäß Anspruch 7, worin die Myocarditis Encephalomyocarditis virale Myocarditis ist.

**Revendications**

**1.** Utilisation d'une chaîne légère d'immunoglobuline libre pour la production d'un médicament thérapeutique pour une maladie cardiaque,
dans laquelle la chaîne légère d'immunoglobuline libre est une chaîne kappa humaine ou un mélange d'une chaîne kappa humaine et d'une chaîne lambda humaine, le mélange ayant un rapport kappa/lambda de 2 ou plus, et dans laquelle la maladie cardiaque est une insuffisance cardiaque ou une myocardite.

**2.** Utilisation selon la revendication 1, dans laquelle la chaîne légère d'immunoglobuline libre est une chaîne kappa.

**3.** Utilisation selon la revendication 1 ou 2, dans laquelle la maladie cardiaque est une myocardite.

**4.** Utilisation selon la revendication 3, dans laquelle la myocardite est une myocardite virale de type encéphalomyocardite.

**5.** Chaîne légère d'immunoglobuline libre pour utilisation dans le traitement d'une maladie cardiaque,
dans laquelle la chaîne légère d'immunoglobuline libre est une chaîne kappa humaine ou un mélange d'une chaîne kappa humaine et d'une chaîne lambda humaine, le mélange ayant un rapport kappa/lambda de 2 ou plus, et dans laquelle la maladie cardiaque est une insuffisance cardiaque ou une myocardite.

**6.** Chaîne légère d'immunoglobuline libre pour utilisation selon la revendication 5, laquelle chaîne légère d'immunoglobuline libre est une chaîne kappa.

**7.** Chaîne légère d'immunoglobuline libre selon la revendication 5 ou 6, dans laquelle la maladie cardiaque est une myocardite.

**8.** Chaîne légère d'immunoglobuline libre selon la revendication 7, dans laquelle la myocardite est une myocardite virale de type encéphalomyocardite.

Fig. 1

## Fig. 2

Free kappa light chain   1 μg/mL

Control group

×400

Fig. 3

Free kappa light chain    1 µg/mL

Control group

×600

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2005114350 A

### Non-patent literature cited in the description

- **Romer F.K. et al.** *Eur. J. Respir. Dis.,* 1984, vol. 65, 292-295
- **Moutsopoulos H.M. et al.** *J. Immunol.,* 1983, vol. 130, 2663-2665
- **Hirohata et al.** *J. Rheumatol.,* 1986, vol. 13, 715-721
- **Fagnart O.C. et al.** *J. Neuroimmunol.,* 1988, vol. 19, 119-132
- **Lamers K.J. et al.** *J. Neuroimmunol.,* 1995, vol. 19, 119-132
- **Contini C. et al.** *J. Neuroimmunol.,* 2000, vol. 108, 221-226
- **Solling K. et al.** *Scan. J. Clin. Lab. Invest.,* 1978, vol. 38, 369-373
- **Solling K. et al.** *Acta Med. Scand.,* 1981, vol. 209, 473-477
- **Cooper A. et al.** *Ann. Rheum. Dis.,* 1968, vol. 27, 537-543
- **Lee D.M. et al.** *Science,* 2002, vol. 297, 1689-1692
- **Kraneveld A.D. et al.** *Proc. Natl. Acad. Sci. USA.,* 2005, vol. 102, 1578-1583
- **Redegeld F.A. et al.** *Nat. Med.,* 2002, vol. 8, 694-701
- **Kishimoto et al.** *J. Pharmacol. Exp. Ther.,* 2001, vol. 299, 645-651
- **Solomon A.** *Methods Enzymol.,* 1985, vol. 116, 101-121
- *J. Immunol. Methods,* 2003, vol. 275, 9-17
- *J. Histochem. Cytochem.,* 1974, vol. 22, 1084-91
- **Matsumori A et al.** *Circulation.,* 1985, vol. 71, 834-839
- **Takahashi, K. et al.** Gen. Virol. Core A-2 antibody, product of Institute of Immunology Co., Ltd, 1992, vol. 73, 667-672